# EUROPEAN PATENT APPLICATION

(11) **EP 4 293 679 A1**
(43) Date of publication of application: **20.12.2023**
(21) Application number: 22179693.1
(22) Date of filing: 17.06.2022
(51) Int. Cl.: G16H 40/20, G16H 50/70

(54) **SYSTEM AND METHOD FOR PREDICTING POSTOPERATIVE BED TYPE**

(71) Applicant: Koninklijke Philips N.V., 5656 AG Eindhoven (NL)
(72) Inventor: DIKIC, Marko, Eindhoven (NL); NIKOLOVA-SIMONS, Mariana, Eindhoven (NL); BOUWMAN, Arthur, 5656AG Eindhoven (NL); COMPAGNER, Wilma, 5656AG Eindhoven (NL)
(74) Representative: Philips Intellectual Property & Standards

(57) **Abstract**

A system and method are provided for generating a predictive model for predicting a postoperative bed type to be used by a patient after surgery. The predictive model is trained on features extracted from medical data and using a postoperative bed type as prediction target in the training. The predictive model is configured to output a probability on a scale 400 which corresponds to, at its lower end, a prediction of a first postoperative bed type and, at its upper end, a prediction of a second postoperative bed. A hybrid model is generated which applies a lower 410 and an upper threshold 420 to the probability scale. If the output probability of the predictive model is in between both thresholds, an expert selection of the bed type is recommended, while otherwise, the prediction of the predictive model is output. The values of the thresholds are optimized using a performance metric.

## Description

### FIELD OF THE INVENTION

The invention relates to a system and computer-implemented method for generating a predictive model for predicting a postoperative bed type for use by a patient after surgery. The invention further relates to a system and computer-implemented method for using a predictive model to predict a postoperative bed type for use by a patient after surgery. The invention further relates to a computer-readable medium comprising instructions to perform any or both of the computer-implemented methods.

### BACKGROUND OF THE INVENTION

Hospitals and other health care institutions are facing capacity challenges, for example due to increased demand for care and shortage of healthcare workers. It is therefore of importance that resources in hospitals and in other health care institutions are efficiently used. To ensure the efficient use of these resources, it is common to plan the use of the resources in advance to ensure their timely availability for patients.

In particular, in such resource planning, the type of bed to be used by a patient after surgery may be predicted to determine which type of bed will be allocated at which time. Such a bed is also referred to as a postoperative bed. Different types of postoperative beds indicate that different level of care is provided to the patient. For example, a higher level of care may be provided to a patient in an intensive care unit (ICU) bed than in a post-anaesthesia care unit (PACU) bed. Another example is that a higher level of care may be provided to a patient in a PACU bed than in a general ward (GW) bed.

However, the erroneous prediction of the postoperative bed type may have detrimental effects, not only for planning purposes but also for the health of patients. For example, if due to erroneous predictions, all non-occupied ICU beds are allocated to patients undergoing or awaiting surgery, elective surgeries may have to be postponed even though sufficient ICU beds would have been available if the postoperative bed type would have been correctly predicted. It is therefore desirable to obtain a predictive model which more accurately predicts a postoperative bed type for use by a patient after surgery.

### SUMMARY OF THE INVENTION

In a first aspect of the invention, a system is provided for generating a predictive model for predicting a postoperative bed type for use by a patient after surgery. The system comprises:
- an input interface for accessing medical data comprising records of surgeries, wherein a record of a surgery is indicative of a postoperative bed type used by a patient after the surgery, wherein the postoperative bed type is one of at least two possible bed types, wherein the medical data comprises data characterizing the surgery and the patient;
- a processor subsystem configured to generate a predictive model for predicting the postoperative bed type to be used by a patient after surgery by:
- training a predictive model on the medical data, wherein the training uses the postoperative bed type as prediction target, wherein the predictive model is configured to output a probability on a scale which corresponds to, at its lower end, a prediction of a first one of the at least two possible bed types and, at its upper end, a prediction of a second one of the at least two possible bed types;
- generating a hybrid predictive model by establishing an upper threshold and a lower threshold within or at an endpoint of the scale, wherein the hybrid predictive model is configured to, during use:
   if the probability is below the lower threshold, output as the prediction the first one of the at least two possible bed types;
   if the probability is above the upper threshold, output as the prediction the second one of the at least two possible bed types;
   if the probability is in between the lower threshold and the upper threshold, recommend or refer to an expert selection of the bed type,
   wherein generating the hybrid model comprises selecting the upper threshold and the lower threshold to optimize a performance metric using the postoperative bed type indicated by the medical data as prediction target.

In a further aspect of the invention, a computer-implemented method is provided for generating a predictive model for predicting a postoperative bed type for use by a patient after surgery. The method comprises:
- accessing medical data comprising records of surgeries, wherein a record of a surgery is indicative of a postoperative bed type used by a patient after the surgery, wherein the postoperative bed type is one of at least two possible bed types, wherein the medical data comprises data characterizing the surgery and the patient;
- generating a predictive model for predicting the postoperative bed type to be used by the patient after surgery by:
- training a predictive model on the medical data, wherein the training uses the postoperative bed type as prediction target, wherein the predictive model is configured to output a probability on a scale which corresponds to, at its lower end, a prediction of a first one of the at least two possible bed types and, at its upper end, a prediction of a second one of the at least two possible bed types;
- generating a hybrid predictive model by establishing an upper threshold and a lower threshold within or at an endpoint of the scale, wherein the hybrid predictive model is configured to, during use:
   if the probability is below the lower threshold, output as the prediction the first one of the at least two possible bed types;
   if the probability is above the upper threshold, output as the prediction the second one of the at least two possible bed types;
   if the probability is in between the lower threshold and the upper threshold, recommend or refer to an expert selection of the bed type,
   wherein generating the hybrid model comprises selecting the upper threshold and the lower threshold to optimize a performance metric using the postoperative bed type indicated by the medical data as prediction target.

In a further aspect of the invention, a system is provided for using a predictive model for predicting a postoperative bed type for use by a patient after surgery. The system comprises:
- an input interface for accessing:
- a predictive model for predicting the postoperative bed type to be used by the patient after surgery, wherein the predictive model is configured for outputting a probability on a scale which corresponds to, at its lower end, a prediction of a first one of the at least two possible bed types and, at its upper end, a prediction of a second one of the at least two possible bed types;
- values for an upper threshold and a lower threshold within or at an endpoint of the scale;
- input data characterizing a planned surgery of the patient and/or characterizing the patient;
- a processor subsystem which is configured to use the predictive model with the values for the upper threshold and the lower threshold to predict a postoperative bed type for use by a patient after surgery by:
- using the input data as input to the predictive model to obtain a probability;
- if the probability is below the lower threshold, output as the prediction the first one of the at least two possible bed types;
- if the probability is above the upper threshold, output as the prediction the second one of the at least two possible bed types; and
- if the probability is in between the lower threshold and the upper threshold, recommend or refer to an expert selection of the bed type.

In a further aspect of the invention, a computer-implemented method is provided for predicting a postoperative bed type for use by a patient after surgery. The method comprises:
- accessing:
- a predictive model for predicting the postoperative bed type to be used by the patient after surgery, wherein the predictive model is configured to output a probability on a scale which corresponds to, at its lower end, a prediction of a first one of the at least two possible bed types and, at its upper end, a prediction of a second one of the at least two possible bed types;

- values for an upper threshold and a lower threshold within or at an endpoint of the scale;
- input data characterizing a planned surgery of the patient and/or characterizing the patient;
- using the predictive model with the values for the upper threshold and the lower threshold to predict a postoperative bed type for use by the patient after surgery by:
- using the input data as input to the predictive model to obtain a probability;
- if the probability is below the lower threshold, output as the prediction the first one of the at least two possible bed types;
- if the probability is above the upper threshold, output as the prediction the second one of the at least two possible bed types; and
- if the probability is in between the lower threshold and the upper threshold, recommend or refer to an expert selection of the bed type.

In a further aspect of the invention, a transitory or non-transitory computer-readable medium is provided, the computer-readable medium comprising data representing a computer program comprising instructions for causing a processor system to perform one or more of the methods described in this specification.

The above aspects of the invention involve generating and using a predictive model for predicting a postoperative bed type for use by a patient. In some aspects of the invention, the predictive model may be generated and used by one and the same system and/or method, while in other aspects of the invention, the predictive model may be generated by a system and/or method which is different from the system and/or method which subsequently uses the predictive model for prediction purposes.

The predictive model may be generated using medical data which comprises records of surgeries. Such a record may for example be a record in a database pertaining to a particular surgery but may also be a row item in a log of surgeries. Typically, the records may pertain to actual surgeries having been performed in the past, e.g., in a hospital or other health care institution. The records may for example be specific to one particular hospital. For each or at least a subset of the surgeries, the medical data may indicate a postoperative bed type which was used by the respective patient after the surgery. The bed type may be one of at least two possible types. For example, the bed type may either be an ICU or a PACU bed. The type of bed may for example be indicated by one or more categorizations and/or by one or more labels. The medical data may further characterize the surgery and the patient. Such characterization may involve characterizing at least one aspect of the surgery, such as for example a type or urgency of the surgery, and at least one aspect of the patient, such as for example an age or a body mass index (BMI) of the patient.

In accordance with the above measures, a machine learning (ML) predictive model is provided and trained to predict the postoperative bed type. The postoperative bed type as indicated in the medical data may be used as prediction target while the characterization of the surgery and/or the patient may be used as feature input(s). Such features may be manually selected from the medical data or may be automatically selected, for example using a univariate and/or multivariate inferential analysis. The predictive model itself may be trained using known machine learning techniques. For example, a gradient boosting model such as CatBoost may be used.

As a result of the training, a classification predictive model is obtained which is capable of predicting the postoperative bed type which will be needed for a patient. The input data may characterize the patient and the planned surgery. The prediction obtained by the predictive model may be interpreted as a recommendation for the postoperative bed type to be used for the patient. Specifically, the output of the predictive model be a probability score, which may assume a value on a probability scale, for example having a range of [0,1] or 0%-100%. One end of the scale may represent a prediction of one type of postoperative bed and at the other end of the scale a prediction of another type of postoperative bed. In a specific example, a prediction score of 0.0 may represent a prediction of an ICU bed while a prediction score of 1.0 may represent a prediction of a PACU bed while a score in-between 0.0 and 1.0 may represent an intermediate probability (e.g., a prediction score of 0.5 representing an even chance between the patient needing an ICU or PACU bed).

Having generated the predictive model, a hybrid model is generated which combines the output of the predictive model and an expert's recommendation as follows. It is noted that this hybrid model is also a predictive model and may also be referred to as such. An upper threshold and a lower threshold are established within or at an endpoint of the scale. If the probability is below the lower threshold, the hybrid model may output as the prediction the bed type which corresponds with the lower endpoint of the scale, if the probability is above the upper threshold, the hybrid model may output as the prediction the bed type which corresponds with the upper endpoint of the scale, and if the probability is in between the lower threshold and the upper threshold, the hybrid model may recommend or refer to an expert selection of the bed type. Effectively, in the region between both thresholds, a user may be referred to the expert selection of the bed type, while outside of this region, the prediction of the predictive model may be used as an output. The expert selection may represent domain knowledge, e.g., in a particular surgical domain, and may be obtained by for example a manual selection by a clinician but also as a prediction from another model, e.g., a so-called expert model. Such an expert model may for example represent a translation of expert domain knowledge on the selection of the postoperative bed type into a computer-readable form. Typically, the expert model is a non-machine learning model.

The values of the thresholds of the hybrid model may be optimized when the hybrid model is generated, namely by selecting the thresholds to optimize a performance metric using the postoperative bed type as prediction target. In other words, the thresholds may be chosen such as the best performance is obtained in accordance with a performance metric. For that purpose, the medical data may, in addition to the actual bed type used by the patient, also comprise a clinician's prediction of the bed type at that time, which may or may not correspond to the actual bed type. This allows the threshold optimization to take into account whether the expert selection is correct or not, and in particular, which types of prediction errors (e.g., false negatives) are common and which types of prediction errors (e.g., false positives) are less common.

The hybrid model may then be output, e.g., in the form of model data defining the predictive model and threshold data defining the thresholds, to be used to predict the postoperative bed type. For example, the hybrid model may be used in a surgical planning department to plan allocation of beds to be used after surgery in a hospital. During use, a system using the hybrid model may for example display either "ICU bed recommended" or "PACU bed recommended" or "Consult clinician's recommendation" for a new patient. In some examples, the expert selection may be available to the system and/or the hybrid model itself and may be output accordingly when the probability generated by the predictive model falls between the lower threshold and the upper threshold. In such examples, the system may indicate whether a particular bed type is predicted by the predictive model or is an expert selection.

The above measures have the advantage that the type of postoperative bed to be used for a patient may be more accurately predicted than conventionally, e.g., by expert selection alone. Interestingly, the inventors have seen that machine learning predictive models may not always improve upon an expert's selection. For example, if the probability score does not indicate a clear preference for either bed type, it may be preferable to refer to an expert selection instead of using the predictive model's output. The hybrid model as described above incorporates this insight by employing thresholds to combine the trained predictive model with the expert selection. The thresholds themselves are optimized during the training for the particular type of medical data, to take into account that the strength and weakness of both the predictive model and the expert selection may differ, e.g., for different medical specialties, for different hospitals, etc. Experiments show that the hybrid model achieves better results compared to only using the predictive model or only the expert selection. Advantageously, more accurate surgery planning may safeguard the health of patients, for example by ensuring that there are sufficient resources available for acute surgeries, or by avoiding that elective surgeries have to be postponed due to a presumed lack of resources.

Optionally, the at least two postoperative bed types differ in level of care provided to the patient. For example, the at least two bed types may comprise an ICU bed type and a PACU bed type, with the former providing a higher level of care than the latter. Another example is that the at least two bed types may comprise an ICU bed type and a general ward (GW) type, with the former providing a higher level of care than the latter. Yet another example is that the at least two bed types may comprise a PACU bed type and a GW bed type, with the former providing a higher level of care than the latter.

The following optional aspect may refer to the system for generating the predictive model, but may, unless otherwise precluded for technical reasons, also represent optional aspects of the corresponding computer-implemented method in which the system's functionality is a method step, and optional aspects of a system and/or a computer-implemented method for using the predictive model for prediction purposes.

Optionally, the performance metric penalizes a first type of erroneous prediction by which a lower level of care bed type is predicted more than a second type of erroneous prediction by which a higher level of care bed type is predicted. In case the prediction of PACU bed type corresponds to 1 or 100% on the probability scale and the prediction of an ICU bed type corresponds to 0 or 0% on the probability scale, the first type of error may be known as a type 1 error (i.e., a false positive prediction) by which a lower level of care bed type is predicted (e.g., predicted bed = PACU, actual bed = ICU) while the second type of erroneous prediction may be known as a type 2 error (i.e., a false negative prediction) by which a higher level of care bed type is predicted (e.g., predicted bed = ICU, actual bed = PACU). When predicting the postoperative bed type to be used, the impact of erroneous predictions may differ between the different types of bed predictions. Namely, if a patient is erroneously predicted to require a higher level of care, e.g., ICU, while in actuality a lower level of care suffices, e.g., PACU, the main impact of this erroneous prediction is suboptimal use of resources and additional financial cost (as a higher level of care may be more costly). However, if a patient is erroneously predicted to require a lower level of care, e.g., PACU or GW, while in actuality a higher level of care is needed, e.g., ICU, this may pose a health risk, as the higher level of care bed may be unavailable since it was not planned. In other words, erroneously predicting a lower level of care bed type may pose a greater health risk than erroneously predicting a higher level of care. To take this asymmetry into account, the performance metric may penalize the former type of erroneous prediction more than the latter. This way, health risks to the patient due to erroneous predictions may be reduced.

Optionally, the performance metric rewards minimization of occurrences of the first type of erroneous prediction while maintaining occurrences of the second type of erroneous prediction below an acceptability threshold. When determining optimal values for the thresholds, it may be desirable to minimize erroneous predictions of a lower level of care bed type, e.g., for the aforementioned health-related reasons. However, when only optimizing for this aspect, this may cause the hybrid model to systematically predict a higher level of care bed type, e.g., to tend towards always predicting an ICU bed, which is also disadvantageous as higher level of care bed types may be scarce and costly resources. By maintaining the occurrences of erroneous predictions of a higher level of care bed type below a threshold, such systematic biases may be avoided or reduced.

Optionally, selecting the upper threshold and the lower threshold comprises one of:
- evaluating different combinations of values for the upper threshold and the lower threshold;
- selecting the lower threshold to be equal to the upper threshold and evaluating different values for both the lower threshold and the upper threshold;
- selecting the lower threshold at the lower endpoint of the scale and evaluating different values for the upper threshold; and
- selecting the upper threshold at the upper endpoint of the scale and evaluating different values for the lower threshold.

Optionally, the processor subsystem is further configured to:
- use a feature extraction technique to identify a set of features in the medical data, which set of features is predictive of the postoperative bed type; and
- train the predictive model using the set of features as an input.

Optionally, the processor subsystem is further configured to:
- receive an identification of a subset of records in the medical data, wherein in surgeries represented by the subset of records, the postoperative bed type is determined by external factors to be disregarded by the predictive model;
- determine if the medical data excluding the subset of records is sufficient for training the predictive model;
- if the medical data excluding the subset of records is determined not to be sufficient for the training of the predictive model:
- train the predictive model on the medical data including the subset of records;
- when selecting the upper threshold and the lower threshold to optimize the performance metric, exclude the subset of records from the medical data.

There may be situations in which the postoperative bed type may be determined by external factors other than entirely medical factors. For example, the time of day a surgery is scheduled at may influence the bed type. A specific example is that a PACU unit may only receive patients after morning surgeries and may discharge those patients to downstream care units in the evening of the same day, since a PACU unit may be closed during the night. It may be preferable to exclude surgeries in which the postoperative bed type is determined by such external factors from the training of the predictive model. However, it may be impossible to distinguish those surgeries in which the postoperative bed type was determined mainly by external factors in spite of medical factors, while excluding all surgeries with possible external factor interference, such as all afternoon surgeries, may result in insufficient training data for the predictive model. Accordingly, the training of the predictive model may nevertheless use the records of such surgeries, but these records may be excluded from the optimization of the thresholds. This way, it may be ensured that thresholds are optimized on a set of surgeries where the influence of the external factors is minimized, leading to a more reliable assessment of the performance. At the same time, the records of surgeries affected by external factors may still be suitable for training the predictive model as the predictive model may learn to predict the impact of those external factors input features are provided to the model which are predictive of the presence and/or type of such factors. For example, as also elucidated below, the time at which a surgery took place may be provided to the predictive model as input feature during training.

Optionally, the processor subsystem is configured to receive the identification of the subset of records in form of a time range in which, or a time after or before which, a respective surgery is performed. This way, surgeries which take place in a specific time range or at a specific time and in which the selection of postoperative bed type may be at least partially determined by external factors may be identified to the system. For example, afternoon surgeries may be identified to the system so that they can be included in the training of the predictive model if otherwise insufficient training data would be available, yet excluded in the optimization of the thresholds.

Optionally, the performance metric is a user-definable metric.

Optionally, the expert selection is a selection by a clinician.

It will be appreciated by those skilled in the art that two or more of the embodiments, implementations, and/or optional aspects of the invention may be combined in any way deemed useful.

Modifications and variations of any system, any computer-implemented method and/or any computer program product, which correspond to the described modifications and variations of another one of said entities, can be carried out by a person skilled in the art on the basis of the present description.

### BRIEF DESCRIPTION OF THE DRAWINGS

These and other aspects of the invention will be apparent from and elucidated further with reference to the embodiments described by way of example in the following description and with reference to the accompanying drawings, in which
Fig. 1 shows an exemplary system for generating and/or using a predictive model for predicting a postoperative bed type, which system comprises an input interface for accessing medical data, a processor subsystem for generating and/or using the predictive model and a user interface subsystem for displaying a graphical user interface to a user;
Fig. 2 shows an error/confusion matrix for the recommendation of a postoperative bed type by a clinician, with the postoperative bed type being either an ICU or a PACU bed;
Fig. 3 illustrates surgeries for which a type 1 error occurred in the clinician's recommendation, i.e., a PACU bed was recommended but the patient ended up in an ICU bed, together with the cumulative percentage of such surgeries depending on the time slot during the day when the surgery occurred;
Fig. 4 shows an error/confusion matrix for the prediction of the postoperative bed type by a predictive model which was trained to predict the postoperative bed type;
Fig. 5 shows a probability scale in which two thresholds are used to select between the prediction of the predictive model and the clinician's recommendation;
Fig. 6 illustrates a selection of thresholds in which false negatives are kept below 10% while optimizing the performance of the hybrid model for a performance metric;
Fig. 7 shows an error/confusion matrix for the prediction of the postoperative bed type by a hybrid model, wherein the hybrid model selects between the prediction of the predictive model and the clinician's recommendation using thresholds;
Figs. 8A and 8B each show an example of a graphical user interface of a system which uses the hybrid model to recommend a postoperative bed type, wherein in the example of Fig. 8A the system recommends a PACU bed while in the example of Fig. 8B the system refers to an expert selection of the postoperative bed type; and
Fig. 9 shows a non-transitory computer-readable medium comprising data.

It should be noted that the figures are purely diagrammatic and not drawn to scale. In the figures, elements which correspond to elements already described may have the same reference numerals.

### List of reference numbers

The following list of reference numbers is provided for facilitating the interpretation of the drawings and shall not be construed as limiting the claims.
- 20: data storage
- 22: medical data
- 24: data representing trained predictive model
- 26: data representing thresholds

- 60: display
- 62: display data
- 80: user input device(s)
- 82: user input data

- 100: system for generating and/or using predictive model
- 120: data storage interface
- 140: processor subsystem
- 142-146: data communication
- 160: memory
- 180: user interface subsystem
- 182: display output interface
- 184: user input interface

- 200: error/confusion matrix of clinician's recommendation
- 202: error/confusion matrix of trained predictive model
- 204: error/confusion matrix of hybrid model
- 210: predicted bed type
- 220: actual bed type
- 300: time
- 310: time interval
- 320: morning surgeries
- 330: percentages of surgeries in time interval or in time intervals lower on the vertical axis

- 400: probability scale
- 410: first threshold
- 420: second threshold

- 500: visualization of prediction performance as a function of threshold values and false negative rate
- 510: prediction performance metric output

- 600: user interface window
- 610: element showing patient data
- 620: element showing surgery data
- 630: element showing output of predictive model
- 640: element showing recommendation
- 642: recommendation by predictive model
- 644: action button to enter clinician's recommendation

- 700: non-transitory computer-readable medium
- 710: data representing computer program

### DETAILED DESCRIPTION OF EMBODIMENTS

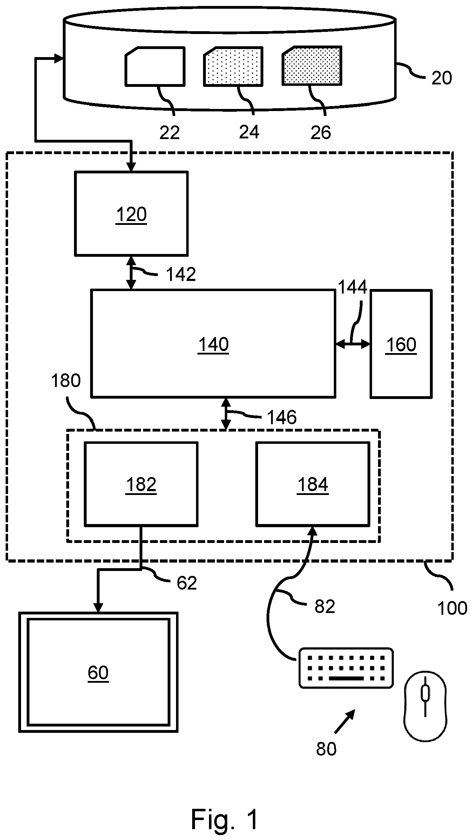

Fig. 1 shows a system 100 which may in some embodiments represent a system for generating a predictive model for predicting a postoperative bed type for use by a patient after surgery, in other embodiments a system for using the predictive model to predict the postoperative bed type for a patient for which a surgery is scheduled, and in yet other embodiments a system for both generating and using the predictive model.

The system 100 is shown to comprise a data storage interface 120 to a data storage 20. In some embodiments, the data storage 20 may store medical data 22 comprising records of surgeries. These records, or other parts of the medical data 22, may characterize a respective surgery and the patient undergoing the surgery. In some embodiments, the data storage 20 may store model data 24 representing a machine learnable predictive model. In some embodiments, the data storage 20 may store threshold data 26 of thresholds described elsewhere in this specification. In general, the data storage 20 may serve as short term storage and/or as long-term data storage. In the example of Fig. 1, the data storage interface 120 is shown to be connected to an external data storage 20, such as a network-accessible data storage 20. Alternatively, the data storage 20 may be an internal data storage of the system 100 (not shown in Fig. 1). In general, the data storage interface 120 may take various forms, such as a hard disk or solid-state disk interface to one or more hard disks and/or solid-state disks, or a network interface to a Local Area Network (LAN) or to a Wide Area Network (WAN). In general, the data storage interface 120 may represent an example of an input interface as described elsewhere in this specification.

The system 100 is further shown to comprise a processor subsystem 140 configured to internally communicate with the data storage interface 120 via data communication 142, with a memory 160 via data communication 144 and with a user interface subsystem 180 via data communication 146. The memory 160 may for example be a volatile memory in which a computer program may be loaded which may cause the processor subsystem 140 to carry out functions which are described in this specification, for example in relation to generating a predictive model and/or to using the predictive model.

In some embodiments, the system 100 may comprise a user interface subsystem 180, which user interface subsystem may be configured to, during operation of the system 100, enable a user to interact with the system 100, for example using a graphical user interface. In particular, as also described elsewhere, the graphical user interface may enable the user to obtain a postoperative bed type recommendation. For that and other purposes, the user interface subsystem 180 is shown to comprise a user input interface 184 configured to receive user input data 82 from one or more user input devices 80 operable by the user. The user input devices 80 may take various forms, including but not limited to a keyboard, mouse, touch screen, microphone, etc. Fig. 1 shows the user input devices to be a keyboard and mouse 80. In general, the user input interface 184 may be of a type which corresponds to the type of user input device(s) 80, i.e., it may be a thereto corresponding type of user device interface. The user interface subsystem 180 is further shown to comprise a display output interface 182 configured to provide display data 62 to a display 60 to visualize output of the system 100. In the example of Fig. 1, the display is an external display 60. Alternatively, the display may be an internal display of the system 100.

In some embodiments, the processor subsystem 140 may be configured to, during operation of the system 100, generate a predictive model for predicting a postoperative bed type for use by a patient after surgery. For that purpose, the processor subsystem 140 may be configured to train a predictive model on the medical data 22 using the postoperative bed type as prediction target. The predictive model may be configured, e.g., by design, to output a probability on a scale which corresponds to, at its lower end, a prediction of a first one of the at least two possible bed types and, at its upper end, a prediction of a second one of the at least two possible bed types. The processor subsystem 140 may be further configured to generate a hybrid predictive model by establishing an upper threshold and a lower threshold within or at an endpoint of the scale. The hybrid predictive model may be configured to, during use, if the probability is below the lower threshold, output as the prediction the first one of the at least two possible bed types, if the probability is above the upper threshold, output as the prediction the second one of the at least two possible bed types, and if the probability is in between the lower threshold and the upper threshold, recommend or refer to an expert selection of the bed type. The processor subsystem 140 may be further configured to, when generating the hybrid model, select the upper threshold and the lower threshold to optimize a performance metric using the postoperative bed type indicated by the medical data as prediction target.

In other embodiments, the processor subsystem 140 may alternatively or additionally be configured to, during operation of the system 100, use the generated predictive model for prediction purposes. For that purpose, the processor subsystem 140 may be configured to access the predictive model as described elsewhere in this specification, values for an upper threshold and a lower threshold as described elsewhere in this specification, and input data characterizing a surgery of a patient and/or characterizing the patient. The processor subsystem 140 may be further configured to use the predictive model with the values for the upper threshold and the lower threshold to predict a postoperative bed type for use by the patient after surgery by using the input data as input to the predictive model to obtain a probability, and if the probability is below the lower threshold, output as the prediction the first one of the at least two possible bed types, if the probability is above the upper threshold, output as the prediction the second one of the at least two possible bed types, and if the probability is in between the lower threshold and the upper threshold, recommend or refer to an expert selection of the bed type.

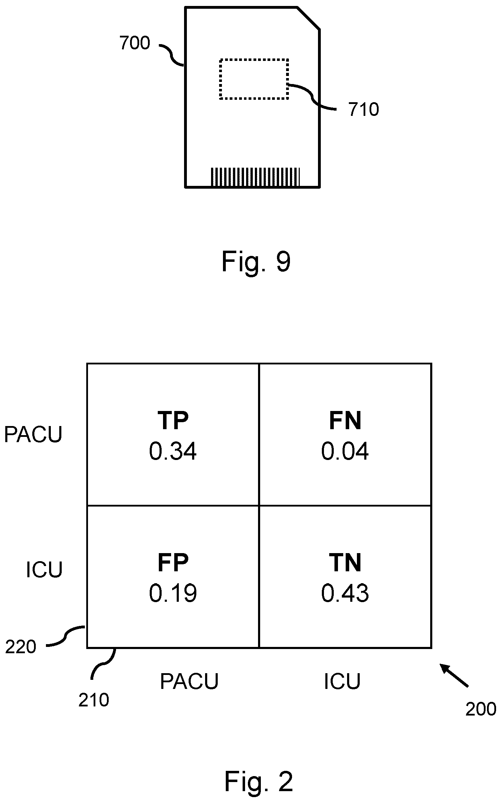

These and other operations of the system 100, and various optional aspects thereof, will be explained in more detail with reference to Fig. 2 et seq.

In general, the system 100 may be embodied as, or in, a single device or apparatus. The device or apparatus may be a general-purpose device or apparatus, such as a workstation or a computer, but may also be application-specific, such as a patient monitor. The device or apparatus may comprise one or more microprocessors which may represent the processor subsystem, and which may execute appropriate software. The software may have been downloaded and/or stored in a corresponding memory, e.g., a volatile memory such as RAM or a non-volatile memory such as Flash. Alternatively, the functional units of the system, e.g., the input interface, the user interface subsystem, and the processor subsystem, may be implemented in the device or apparatus in the form of programmable logic, e.g., as a Field-Programmable Gate Array (FPGA). In general, each functional unit of the system 100 may be implemented in the form of a circuit. It is noted that the system 100 may also be implemented in a distributed manner, e.g., involving different devices or apparatuses. For example, the distribution may be in accordance with a client-server model, e.g., using a server and workstation. For example, the user input interface and the display output interface may be part of the workstation, while the processor subsystem may be a subsystem of the server. It is noted that various other distributions are equally conceivable.

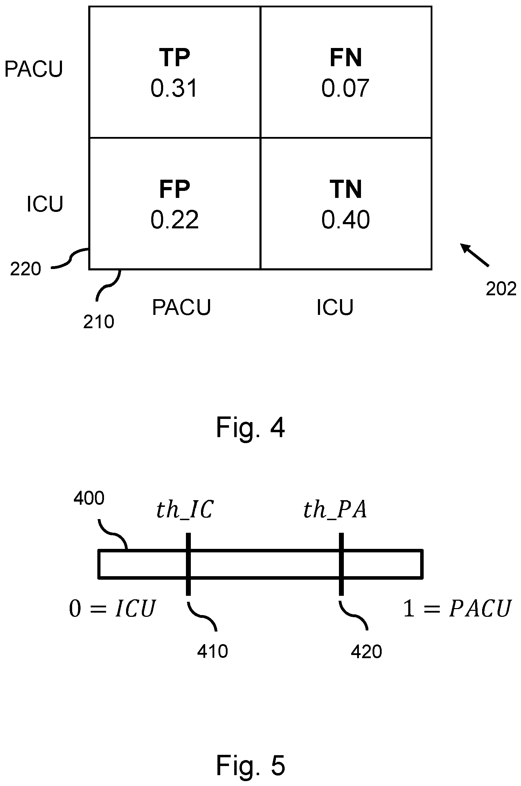

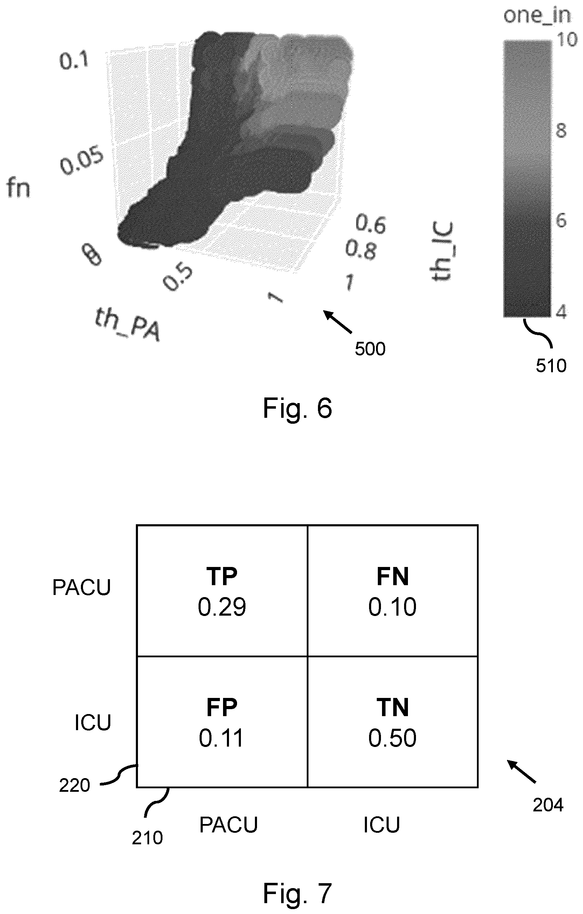

Fig. 2 shows an error/confusion matrix 200 for the recommendation of a postoperative bed type by a clinician, with the postoperative bed type being either an ICU or a PACU bed, which shows along the horizontal axis 210 the predicted bed type and on the vertical axis the actual bed type. This error matrix was generated based on actual medical data of cardiothoracic surgeries, in which both the recommendation of the clinician before the surgery and the actual bed type in which the patient ended up in after surgery were identified and depicted as columns and rows of the confusion matrix, respectively. The values in the error matrix 200, as well as those of other error matrixes described in this specification, are normalized to 1.0 (=100%). It can be seen that for 34% of the patients a PACU bed is predicted by the clinician before surgery, and a PACU bed is indeed sufficient after surgery (sometimes also referred to as 'fast-track'), and that for 43% of the patients an ICU bed is predicted by the clinician before surgery, and an ICU bed is indeed needed after surgery. For the error/confusion matrix 200 of Fig. 2 and that of Figs. 4 and 7, the correct prediction of a PACU bed may be considered as a true positive (TP), while the correct prediction of an ICU bed may be considered as a true negative (TN). However, there are also erroneous predictions in the form of false positives (FP) and false negatives (FN). Namely 19% of the patients ended up in an ICU bed although the clinician recommended a PACU bed (false positive, or type 1 error, a lower level of care was predicted than needed). Similarly, 4% of the patients ended up in a PACU bed although the clinician recommended an ICU bed (false negative, or type 2 error, a higher level of care was predicted).

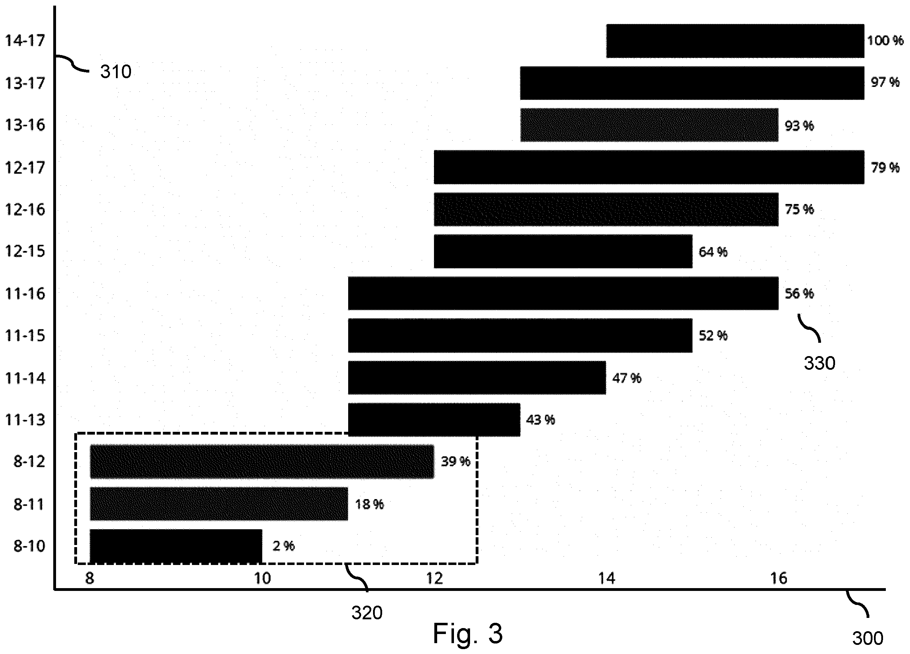

The postoperative bed type may be determined based on medical factors, e.g., the type of surgery and the age and BMI of a patient, as well as external factors such as start and end time of the surgery. Detecting surgeries where external factors were prevailing may lead to more precise analysis of errors and to training of a more precise predictive model. Fig. 3 shows all the cardiothoracic surgeries for which a type 1 error (a lower level of care was predicted than needed) occurred in the clinician's recommendation with respect to the time slot when the surgery was performed. The vertical axis 310 shows different time intervals in which the surgeries were performed, the horizontal axis 300 being a time axis (in hours of the day), and the bars being again a visualization of the time intervals set out on the vertical axis 310. The percentages 330 next to the bars indicate the percentage of all depicted surgeries performed in the respective time slot, or in any other time slot shown lower on the vertical axis 310 and are therefore calculated in a cumulative manner. The PACU unit may be closed at night, and patients who were operated late in the morning or in the afternoon and required at least the level of care provided by in a PACU unit may thus be assigned an ICU bed. Hence, for all the surgeries that were performed late in the morning or in the afternoon, the external factors may prevail in assigning a bed over the medical factors. On the other hand, for morning surgeries, which are indicated in Fig. 3 by the bounding box 320, the choice of ICU bed or PACU bed may be mainly determined by medical factors. The conclusion may be that only 39% of all type 1 error surgeries should be considered as an erroneous recommendation, while the rest only appear to be erroneous predictions, while in reality the recommendation was not followed due to external factors.

A predictive model was trained to predict the postoperative bed type for use by a patient after surgery based on the medical data of the aforementioned cardiothoracic surgeries. In this specific example, records of 2363 cardiothoracic surgeries were accessed, which were filtered down to 1481 cardiothoracic surgeries, e.g., by selecting only elective surgeries, selecting surgeries where the postoperative bed type is specified, etc. Also, by way of the filtering, some surgeries were omitted from which it was certain that the choice of bed type was determined by external factors rather than by medical factors. From these 1481 surgeries, 1049 were used for training the predictive model and 251 for testing purposes. The model itself was a binary classification model which was trained using CatBoost to predict either an ICU bed (prediction target = 0) or a PACU bed (prediction target = 1.0). The features used as input during the training included patient features, such as the ASA score, the number of medications taken by a patient at home, the creatine level, the gender, the BMI, the age group to which the patient belonged, the number of procedures that the patient will undergo, etc., as well as surgery-related features, such as the type of surgery, which for example included aortic valve repairs and coronary artery bypass grafts.

Fig. 4 shows an error matrix 202 for the prediction of the postoperative bed type by the aforementioned predictive model. It can be seen that for the predictive model, for 31% of the patients a PACU bed is predicted by the predictive model, and a PACU bed is sufficient after surgery, and that for 40% of the patients an ICU bed is predicted by the predictive model, and an ICU bed is needed after surgery. However, for 22% of the patients a PACU bed is predicted by the predictive model, but an ICU bed (i.e., a higher level of care than originally predicted) is needed after surgery, and for 7% of the patients the predictive model predicts an ICU bed, but a PACU bed (i.e., a lower level of care than predicted) is sufficient after surgery. When comparing the error matrix 202 of the predictive model with the error matrix 200 of the clinician's recommendation as discussed with reference to Fig. 2, it appears at first glance that the predictive model does not improve upon the clinician's recommendation. This is seemingly confirmed in Table 1, which shows well known performance metrics for classification models in term of Accuracy ((TP+TN)/( TP+TN+FP+FN)), Recall (Sensitivity = TP/(TP+FN)), Precision (PPV=TP/(TP+FP)).

**Table 1: Comparison of clinician's and predictive models' performance**

| Metric | Clinician's recommendation | Predictive model |
|---|---|---|
| Accuracy | 0.77 | 0.71 |
| Recall (Sensitivity) | 0.89 | 0.84 |
| Precision (PPV) | 0.64 | 0.58 |

However, when the predictive model is only evaluated on morning surgeries (being a subset of the test data in which the selection of bed type was considered not or at least less influenced by external factors), an improvement could be seen over the clinician's recommendation in terms of number of patients classified correctly for the PACU bed type. To quantify this improvement, a custom performance metric may be used, which is defined by *(TP* + *FP_{corrected}*)/*FP_{corrected}* with *TP* referring to the number of true positives and *FP_{corrected}* to the number of false positives which were scheduled in the morning, since if a false positive surgery was not scheduled in the morning one may not be certain whether it is a false positive due to medical reasons or due external factors, this is why *FP_{corrected}* may be used rather than *FP.* This performance metric may be considered as a 'one-in' metric which expresses one in how many patients were recommended for PACU but ended up in ICU, which is a prediction error which poses more health risks than the other way around and therefore is more relevant in the assessment of the performance of the prediction.

When applying this 'one-in' performance metric to both the clinician's recommendations as discussed with reference to Fig. 2 and the predictive model as discussed with reference to Fig. 4, an improvement is visible for the surgeries scheduled in the morning, in that for the clinician, approximately 1 in 5 patients was recommended for PACU yet ended up in an ICU bed, while for the predictive model, only 1 in 7 patients was predicted for PACU yet ended up in an ICU bed.

Fig. 5 shows a probability scale 400 associated with the predictive model. Here, the lower endpoint of the scale, which corresponds to a prediction score of 0.0, represents a prediction of an ICU bed while the higher endpoint of the scale, which corresponds to a prediction score of 1.0, represents a prediction of a PACU bed. To further improve on the predictive model's performance, a hybrid predictive model may be generated by establishing an upper threshold 420 and a lower threshold 410 each within or at an endpoint of the scale 400, with the thresholds being used to select whether the prediction of the predictive model is to be followed or whether an expert selection is recommended. In particular, below the lower threshold 410, the predictive model's prediction of an ICU bed may be followed, while above the upper threshold 420, the predictive model's prediction of a PACU bed may be followed, while in-between the lower threshold 410 and the upper threshold 420, a user may be recommended or referred to the expert selection.

To determine optimal values for the upper threshold 420 and the lower threshold 410, a performance metric may be used. This performance metric may be, or may comprise, the aforementioned 'one-in' performance metric to quantify how many patients which were recommended for PACU ended up in an ICU bed. In optimizing the threshold values, the performance metric may be optimized so that the number of patients recommended for PACU yet ending up in an ICU bed is reduced or even minimized.

Fig. 6 illustrates the selection of thresholds, showing a graph 500 in which along one axis a value of the lower threshold (*th_IC*) is set out, along another axis a value of the upper threshold (*th_PA*), and along yet another axis a percentage of false negative predictions (*fn*) (i.e., a type 2 error). In the graph 500, a surface is shown which, for different combinations of thresholds, indicates the percentage of false negative predictions, with the surface being color-coded using the value of the 'one-in' performance metric (*one_in*) for the combination of *th_IC* and *th_PA.* In this example, best results are achieved with *th_IC* = 0.49 and *th_PA* = 0.76, which keeps the percentage of false negatives below 10% (corresponding to < 0.1 in the graph) and obtains the best 'one-in' performance, meaning the lowest misclassification of an PACU bed when it should be an ICU bed.

Fig. 7 shows an error matrix 204 for the prediction of the postoperative bed type by the hybrid model as discussed above. It can be seen that for 29% of the patients a PACU bed is predicted by the hybrid model, and a PACU bed is sufficient after surgery, and that for 50% of the patients an ICU bed is predicted by the hybrid model, and an ICU bed is indeed needed after surgery. However, there are also erroneous predictions. Namely, for 11% of the patients a PACU bed is predicted by the hybrid model, but an ICU bed is actually needed, and for 10% of the patients the hybrid model predicts an ICU bed, but a PACU bed is sufficient after surgery. According to the aforementioned 'one-in' metric, for approximately 1 in 10 patients, a PACU bed was predicted even though the patient ended up in an ICU bed, which is a significant improvement over the 1-in-5 score of the clinician recommendation and 1-in-7 score of the prediction by the predictive model alone. Such improved performance is also demonstrated in Table 2.

**Table 2: Comparison of clinician's and hybrid models performance**

| Metric | Clinician's recommendation | Hybrid model |
|---|---|---|
| Accuracy | 0.77 | 0.79 |
| Recall (Sensitivity) | 0.89 | 0.75 |
| Precision (PPV) | 0.64 | 0.72 |

In alternative embodiments of the hybrid model, both thresholds may be set to the same value and thereby represent one and the same threshold. This way, the optimization may be reduced in complexity as one dimension is removed from the solution space. However, then the clinician's recommendation is excluded as well. In other embodiments, one of both thresholds may be fixed to a respective endpoint of the probability scale. For example, the lower threshold may be set to the lower endpoint of the probability scale while different values for the upper threshold may be evaluated. In this case, the output of the hybrid model will be either PACU if probability > *th_PA* or the clinician's recommendation otherwise. Another example is that the upper threshold may be set to the upper endpoint of the probability scale while different values for the lower threshold may be evaluated. In this case, the output of the hybrid model will be either ICU if probability < *th_IC* or the clinician's recommendation otherwise. Also here, the optimization may be reduced in complexity as one dimension is removed from the solution space. In addition, such embodiments may take into account that the clinician's recommendation may be better than that of the predictive model at one end of the scale.

With continued reference to the features used during the training and subsequent use, such features may be extracted from the medical data, for example using univariate and/or multivariate inferential analysis. Various types of features may be extracted. For example, some types of features may characterize the surgery while other types of features may characterize the patient. Examples of the former type of features are given in Table 3 below, while examples of the latter type of features are given in Table 4 below. It is noted that these features may be considered as potentially relevant features for the prediction of the postoperative bed type in the field of cardiothoracic surgeries, but that in other fields, e.g., in other surgical disciplines, other features may be of relevance. It is further noted that typically, all features are available before the surgery is performed, e.g., at the point of surgery planning, and typically even before the patient is hospitalized.

**Table 3: Features based on surgery characteristics**

| **Features** | | **Examples of values** |
|---|---|---|
| Surgeon ID | unclustered | ID_6791, ID 86 |
| | clustered | - Hierarchical clustering - ID_C1, ID C2 |
| Surgeon type | categorical | Attendings vs. Residents |
| Surgery Procedures (Px) | unclustered | CABG, AVR, etc. |
| | clustered | According to: |
| | | - medical subspecialties |
| | | - Hierarchical clustering - Px_C1, Px_C2 |
| Ave Px time per surgeon | | 183 min, 316 min |
| Number of Px | categorical | Single, double, multiple (>=3) |
| | discrete | 1, 2, 3, 4, 5, ... |
| Surgery urgency | categorical | Acute, Elective |
| Post OR type of bed | categorical | ICU, PACU, general ward (GW) |

**Table 4: Features based on patient characteristics**

| **Features** | | **Examples of values** |
|---|---|---|
| Age | discrete | 67 years, 86 years |
| | categorical | [18-29], [30-44], [45-69], ... |
| Gender | categorical | Male vs. Female |
| BMI | categorical | Underweight, Normal, Overweight, Obese |
| ASA score | discrete | 1, 2, 3, 4, 5 |
| Medication numbers | discrete | 1,2, ...,22 |
| | categorical | [1, 5], [6-10], [11-15], [16++] |
| Creatinine levels | categorical | renal failure, severe decrease, moderate decrease, mild decrease, normal |

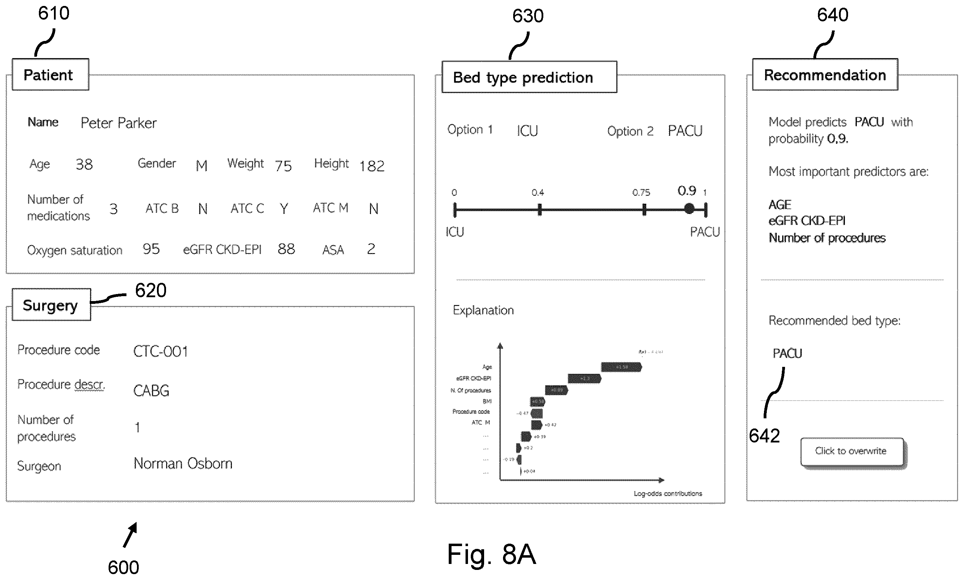

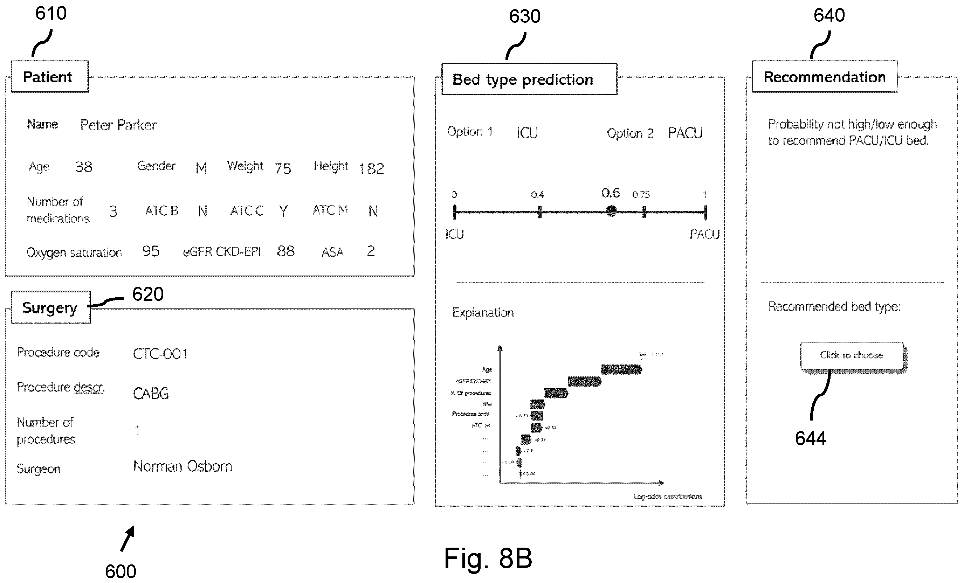

Figs. 8A and 8B each show an example of a graphical user interface 600 of a system which uses the hybrid model to recommend a postoperative bed type. In both figures, the graphical user interface 600 comprises various graphical elements, such as an element 610 which shows information of a patient for which surgery is planned, such as the name, age, gender, number of medications, etc., and an element 620 which shows information about the upcoming surgery, such as the procedure code, procedure description, number of procedures to be performed, surgeon, etc. Some of the information shown in these elements may also be used as feature input to the hybrid model. In element 630, an output of the predictive model is shown, showing the probability scale, the thresholds of the hybrid model (0.4 and 0.75) and the output of the predictive model for this patient and surgery. In the example of Fig. 8A, this probability score is 0.9 which exceeds the hybrid model's upper threshold of 0.75. As such, the hybrid model may follow the predictive model's recommendation, as depicted in element 640, namely a PACU bed is recommended to the user. In some embodiments, the graphical user interface may further indicate the most important predictors for this recommendation, being in this example the age of the patient, the e-GFR (CDK-EPI) value (estimated Glomerular Filtration Rate) and the number of procedures to be carried out during the surgery. In the example of Fig. 8B, the bed prediction probability score is 0.6, which is below the upper threshold of 0.75 but above the lower threshold of 0.4. As such, the hybrid model refers to a recommendation by a clinician. This recommendation may for example be obtained by the system by prompting the user to click on a button 644, which may open an input field in which the clinician's recommendation can be entered or in which the bed type can be chosen, e.g., from PACU or ICU as options. In other examples, the expert's recommendation may already be available to the system or hybrid model and may be output to the user instead of the predictive model's prediction.

It will be appreciated that any method described in this specification may be implemented on a computer as a computer implemented method, as dedicated hardware, or as a combination of both. As also illustrated in Fig. 9, instructions for a computer, e.g., in the form of executable code, may be stored on a computer readable medium 700, e.g., in the form of a series 710 of machine-readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, servers, online software, etc. Fig. 9 shows a memory device 700.

In accordance with an abstract of the present application, a system and method are provided for generating a predictive model for predicting a postoperative bed type to be used by a patient after surgery. The predictive model is trained on features extracted from medical data and using a postoperative bed type as prediction target in the training. The predictive model is configured to output a probability on a scale which corresponds to, at its lower end, a prediction of a first postoperative bed type and, at its upper end, a prediction of a second postoperative bed. A hybrid model is generated which applies a lower threshold and an upper threshold to the probability scale. If the output probability of the predictive model is in between both thresholds, an expert selection of the bed type is recommended, while otherwise, the prediction of the predictive model is output. The values of the thresholds are optimized using a performance metric.

Examples, embodiments or optional features, whether indicated as non-limiting or not, are not to be understood as limiting the invention as claimed.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of' when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A system (100) for generating a predictive model for predicting a postoperative bed type for use by a patient after surgery, comprising:
- an input interface (120) for accessing medical data comprising records of surgeries, wherein a record of a surgery is indicative of a postoperative bed type used by a patient after the surgery, wherein the postoperative bed type is one of at least two possible bed types, wherein the medical data comprises data characterizing the surgery and the patient;
- a processor subsystem (140) configured to generate a predictive model for predicting the postoperative bed type to be used by a patient after surgery by:
- training a predictive model on the medical data, wherein the training uses the postoperative bed type as prediction target, wherein the predictive model is configured to output a probability on a scale (400) which corresponds to, at its lower end, a prediction of a first one of the at least two possible bed types and, at its upper end, a prediction of a second one of the at least two possible bed types;
- generating a hybrid predictive model by establishing an upper threshold (410) and a lower threshold (420) within or at an endpoint of the scale, wherein the hybrid predictive model is configured to, during use:
- if the probability is below the lower threshold, output as the prediction the first one of the at least two possible bed types;
- if the probability is above the upper threshold, output as the prediction the second one of the at least two possible bed types;
- if the probability is in between the lower threshold and the upper threshold, recommend or refer to an expert selection of the bed type,
wherein generating the hybrid model comprises selecting the upper threshold and the lower threshold to optimize a performance metric using the postoperative bed type indicated by the medical data as prediction target.

2. The system (100) according to claim 1, wherein the at least two postoperative bed types differ in level of care provided to the patient.

3. The system (100) according to claim 1 or 2, wherein the at least two bed types comprise:
- an intensive care unit (ICU) bed type and a post-anaesthesia care unit (PACU) bed type;
- an intensive care unit (ICU) bed type and a general ward bed type; or
- a post-anaesthesia care unit (PACU) bed type and a general ward bed type.

4. The system (100) according to claim 2 or 3, wherein the performance metric penalizes a first type of erroneous prediction by which a lower level of care bed type is predicted more than a second type of erroneous prediction by which a higher level of care bed type is predicted.

5. The system (100) according to claim 4, wherein the performance metric rewards minimization of occurrences of the first type of erroneous prediction while maintaining occurrences of the second type of erroneous prediction below an acceptability threshold.

6. The system (100) according to any one of claims 1 to 5, wherein selecting the upper threshold (410) and the lower threshold (420) comprises one of:
- evaluating different combinations of values for the upper threshold and the lower threshold;
- selecting the lower threshold to be equal to the upper threshold and evaluating different values for both the lower threshold and the upper threshold;
- selecting the lower threshold at the lower endpoint of the scale and evaluating different values for the upper threshold; and
- selecting the upper threshold at the upper endpoint of the scale and evaluating different values for the lower threshold.

7. The system (100) according to any one of the above claims, wherein the processor subsystem (140) is further configured to:
- use a feature extraction technique to identify a set of features in the medical data, which set of features is predictive of the postoperative bed type; and
- train the predictive model using the set of features as input.

8. The system (100) according to any one of claims 1 to 7, wherein the processor subsystem (140) is further configured to:
- receive an identification of a subset of records in the medical data, wherein in surgeries represented by the subset of records, the postoperative bed type is determined by external factors to be disregarded by the predictive model;
- determine if the medical data excluding the subset of records is sufficient for training the predictive model;
- if the medical data excluding the subset of records is determined not to be sufficient for the training of the predictive model:
- train the predictive model on the medical data including the subset of records;
- when selecting the upper threshold and the lower threshold to optimize the performance metric, exclude the subset of records from the medical data.

9. The system (100) according to claim 8, wherein the processor subsystem (140) is configured to receive the identification of the subset of records in form of a time range in which, or a time after or before which, a respective surgery is performed.

10. The system (100) according to any one of claims 1 to 9, wherein the performance metric is a user-definable metric.

11. A system (100) for using a predictive model for predicting a postoperative bed type for use by a patient after surgery, comprising:
- an input interface (120) for accessing:
- a predictive model for predicting the postoperative bed type to be used by the patient after surgery, wherein the predictive model is configured for outputting a probability on a scale (400) which corresponds to, at its lower end, a prediction of a first one of the at least two possible bed types and, at its upper end, a prediction of a second one of the at least two possible bed types;
- values for an upper threshold (410) and a lower threshold (420) within or at an endpoint of the scale;
- input data characterizing a planned surgery of the patient and/or characterizing the patient;
- a processor subsystem (140) which is configured to use the predictive model with the values for the upper threshold and the lower threshold to predict a postoperative bed type for use by a patient after surgery by:
- using the input data as input to the predictive model to obtain a probability;
- if the probability is below the lower threshold, output as the prediction the first one of the at least two possible bed types;
- if the probability is above the upper threshold, output as the prediction the second one of the at least two possible bed types; and
- if the probability is in between the lower threshold and the upper threshold, recommend or refer to an expert selection of the bed type.

12. The system (100) according to claim 11, wherein the expert selection is a selection by a clinician.

13. A computer-implemented method for generating a predictive model for predicting a postoperative bed type for use by a patient after surgery, comprising:
- accessing medical data comprising records of surgeries, wherein a record of a surgery is indicative of a postoperative bed type used by a patient after the surgery, wherein the postoperative bed type is one of at least two possible bed types, wherein the medical data comprises data characterizing the surgery and the patient;
- generating a predictive model for predicting the postoperative bed type to be used by the patient after surgery by:
- training a predictive model on the medical data, wherein the training uses the postoperative bed type as prediction target, wherein the predictive model is configured to output a probability on a scale which corresponds to, at its lower end, a prediction of a first one of the at least two possible bed types and, at its upper end, a prediction of a second one of the at least two possible bed types;
- generating a hybrid predictive model by establishing an upper threshold and a lower threshold within or at an endpoint of the scale, wherein the hybrid predictive model is configured to, during use:
- if the probability is below the lower threshold, output as the prediction the first one of the at least two possible bed types;
- if the probability is above the upper threshold, output as the prediction the second one of the at least two possible bed types;
- if the probability is in between the lower threshold and the upper threshold, recommend or refer to an expert selection of the bed type,
wherein generating the hybrid model comprises selecting the upper threshold and the lower threshold to optimize a performance metric using the postoperative bed type indicated by the medical data as prediction target.

14. A computer-implemented method for predicting a postoperative bed type for use by a patient after surgery, comprising:
- accessing:
- a predictive model for predicting the postoperative bed type to be used by the patient after surgery, wherein the predictive model is configured to output a probability on a scale which corresponds to, at its lower end, a prediction of a first one of the at least two possible bed types and, at its upper end, a prediction of a second one of the at least two possible bed types;
- values for an upper threshold and a lower threshold within or at an endpoint of the scale;
- input data characterizing a planned surgery of the patient and/or characterizing the patient;
- using the predictive model with the values for the upper threshold and the lower threshold to predict a postoperative bed type for use by the patient after surgery by:
- using the input data as input to the predictive model to obtain a probability;
- if the probability is below the lower threshold, output as the prediction the first one of the at least two possible bed types;
- if the probability is above the upper threshold, output as the prediction the second one of the at least two possible bed types; and
- if the probability is in between the lower threshold and the upper threshold, recommend or refer to an expert selection of the bed type.

15. A transitory or non-transitory computer-readable medium (700) comprising data (710) representing a computer program, the computer program comprising instructions for causing a processor system to perform the method according to claim 13 or 14.
